# EUROPEAN PATENT SPECIFICATION

(11) **EP 4 606 791 B1**
(45) Date of publication and mention of the grant of the patent: **08.04.2026**
(21) Application number: 24158915.9
(22) Date of filing: 21.02.2024
(51) Int. Cl.: C07D 209/18, C07C 243/38, C07D 209/26

(54) **IMPROVED PROCESS FOR THE PREPARATION OF INDOMETHACIN**
VERBESSERTES VERFAHREN ZUR HERSTELLUNG VON INDOMETHACIN
PROCÉDÉ AMÉLIORÉ POUR LA PRÉPARATION D'INDOMÉTHACINE

(43) Date of publication of application: 27.08.2025
(73) Proprietor: F.I.S.- Fabbrica Italiana Sintetici S.p.A., 36075 Montecchio Maggiore (VI) (IT)
(72) Inventor: Venturino, Federico, 36075 Montecchio Maggiore (VI) (IT); Semeraro, Floriana, 36075 Montecchio Maggiore (VI) (IT); Stabile, Paolo, 36075 Montecchio Maggiore (VI) (IT); Gesualdi, Fausto Gaetano, 36075 Montecchio Maggiore (VI) (IT)

(56) References cited:
- CN-A- 115 925 608
- RUSU D ET AL: "The control over the new obtaining procedeum of indomethacin", JOURNAL OF PHARMACEUTICAL AND BIOMEDICAL ANALYSIS, vol. 17, no. 3, July 1998 (1998-07-01), AMSTERDAM, NL, pages 409 - 413, XP093193471, ISSN: 0731-7085, DOI: 10.1016/S0731-7085(97)00209-4
- MAGUIRE ANITA R. ET AL: "Synthesis of indomethacin analogues for evaluation as modulators of MRP activity", vol. 9, no. 3, March 2001 (2001-03-01), AMSTERDAM, NL, pages 745 - 762, XP093193477, ISSN: 0968-0896, Retrieved from the Internet <URL:https://pdf.sciencedirectassets.com/271397/1-s2.0-S0968089600X00892/1-s2.0-S0968089600002923/main.pdf?X-Amz-Security-Token=IQoJb3JpZ2luX2VjEB0aCXVzLWVhc3QtMSJHMEUCIAEYSo5gTAI1wNCQXKQQXrCkAT59E7nOxxmGq6iATU2rAiEAvL9bBcKoPU71rrCQsdbQ/22bUOk8Wee15Z+nj0FCx6wqswUIFRAFGgwwNTkwMDM1NDY4NjUiDFWjwj7+qovoCL3Tv> DOI: 10.1016/S0968-0896(00)00292-3
- KEVIN R. CAMPOS ET AL: "A General Synthesis of Substituted Indoles from Cyclic Enol Ethers and Enol Lactones", ORGANIC LETTERS, vol. 6, no. 1, 2004, pages 79 - 82, XP055123611, ISSN: 1523-7060, DOI: 10.1021/ol036113f

## Description

### Technical Field

The present invention refers to an improved process for the preparation of Indomethacin.

### Background Art

Indomethacin is a nonsteroidal anti-inflammatory drug (NSAID) commonly used as a prescription medication to reduce fever, pain, stiffness, and swelling from inflammation. It works by inhibiting the production of prostaglandins, endogenous signaling molecules known to cause these symptoms.

Indomethacin was discovered and approved for medical use in the early 1960s. It is on the World Health Organization's List of Essential Medicines.

Indomethacin is an indole-3-acetic acid wherein the indole ring is substituted at positions 1, 2 and 5 by p-chlorobenzoyl, methyl, and methoxy groups, respectively. Its chemical name is 2-[1-(4-chlorobenzoyl)-5-methoxy-2-methylindol-3-yl]acetic acid and has the following chemical formula (I):

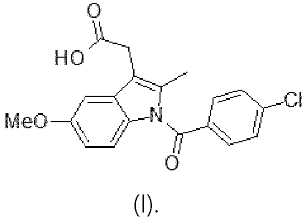

Indomethacin can be prepared according to several synthetic processes described in the literature.

The first synthesis of Indomethacin is described for example in J. Am. Chem. Soc. 1963, pages 488-489. The route of synthesis is attached in the following page. The starting material 5-methoxy-2-methylindole-3-acetic acid A is converted into its anhydride by reaction with dicyclohexylcarbodiimide. The latter is then treated with zinc chloride and t-butanol to give t-butyl-5-methoxy-2-methyl-3-indolylacetate B. Indomethacin is then obtained by acylation of the t-butyl ester with p-chlorobenzoyl chloride followed by pyrolysis of the resulting intermediate C at 210°C.

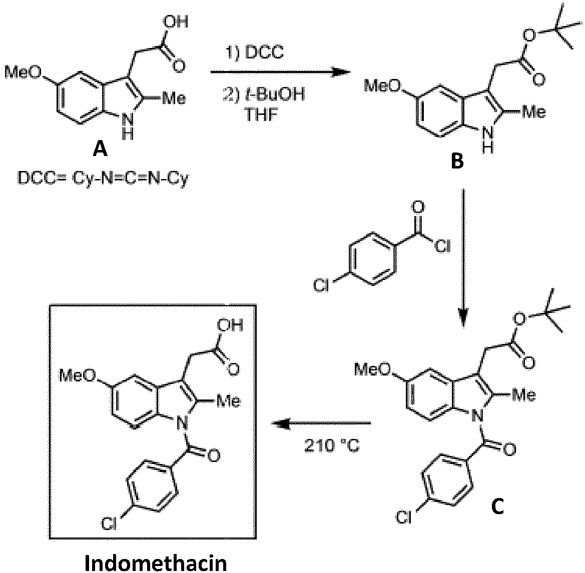

A new route of synthesis towards Indomethacin was later developed (J. Pharm. Biomed. Anal. 1998, 17, pages 409-413), comprising the following steps:

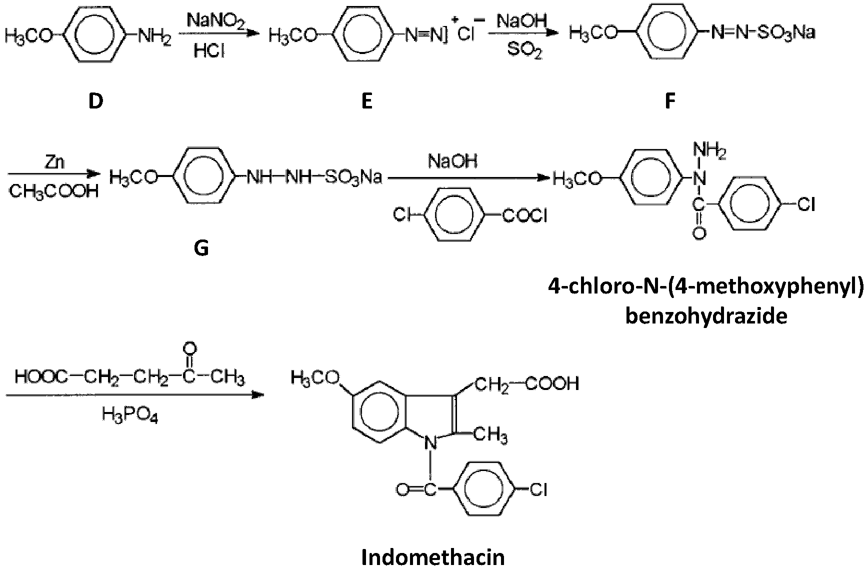

According to this new method, p-anisidine D is reacted with sodium nitrite to give the corresponding diazonium salt E, which is in turn converted into the diazensulfonate F with sulfur dioxide under basic conditions. The following reduction with zinc in acetic acid produces the hydrazine sulfonate G. By reaction with p-chlorobenzoyl chloride and concomitant desulfonation the intermediate 4-chloro-N-(4-methoxy phenyl)benzohydrazide is then obtained. This intermediate is finally reacted with levulinic acid (or 4-oxopentanoic acid) in the presence of phosphoric acid to achieve Indomethacin. This route of synthesis has many advantages with respect to the previous synthetic approach, such as higher yields.

Other processes later developed for the preparation of Indomethacin, reported for example in J. Pharm. Biomed. Anal. 2000, 24, 19-24, in Bioorg. Med. Chem. 2001, 9,745-762 or in Org. Letters 2004, 6, 79-82, have in common the intermediate 4-chloro-N-(4-methoxyphenyl)benzo hydrazide as final intermediate for the preparation of Indomethacin.

Modern synthetic approaches for the industrial manufacturing of Indomethacin, therefore, are based on the key intermediate 4-chloro-N-(4-methoxyphenyl) benzohydrazide of formula (II):

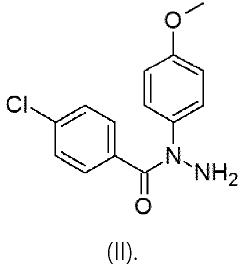

Since in all the synthetic routes where said intermediate is employed it is the last one before the obtainment of the final product Indomethacin, it clearly follows that its purity can affect final product impurity profile. For this reason, regulatory guidelines are nowadays strongly recommending developing manufacturing processes for Active Pharmaceutical Ingredients (APIs) in which the impurities are suitably controlled before the final synthetic step.

Among the several organic impurities specified for Indomethacin in the European Pharmacopoeia (European Pharmacopoeia 11.0, pages 3077-3079), 4-chloro-N-(4-methoxyphenyl) benzamide of formula (III): 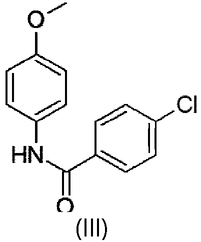 is identified as Impurity C and 4-chloro-N'-(4-chlorobenzoyl)-N-(4-methoxyphenyl)benzo hydrazide of formula (IV): 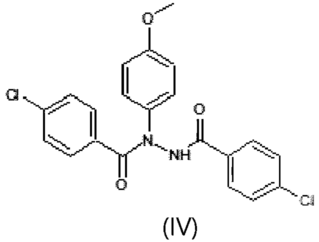 is identified as Impurity F. Both the impurities are typical process impurities generated in the synthetic process, which are normally found in the key intermediate 4-chloro-N-(4-methoxyphenyl)benzohydrazide of formula (II) within the range 1%-15% A/A% as determined by HPLC.

Such a variability on the impurity content of the key intermediate of formula (II) represents an evident issue in terms of reproducibility and control of the key process intermediate quality and, consequently, of the final product Indomethacin.

Impurities of formula (III) and formula (IV), in fact, together with other unspecified organic impurities eventually found in intermediate of formula (II), can be carried forward to the final API Indomethacin when intermediate of formula (II) is converted into Indomethacin, thus affecting final product impurity profile.

Prior art methods described for the preparation of Indomethacin through the key intermediate benzohydrazide of formula (II) do not provide an adequate control on process impurities. Impurity of formula (III) and impurity of formula (IV), in fact, are typically found in the intermediate of formula (II) ranging from 1% to 15% A/A% as determined by HPLC. Further reference may be made to CN115925608.

Thus, there is a need for a process for preparing Indomethacin characterized by a proper control of organic impurities content.

### Summary of the invention

The problem addressed by the present invention is therefore that of providing a process for the preparation of Indomethacin, which allows to control Indomethacin impurity content, in particular the content of impurity of formula (III) and impurity of formula (IV).

This problem is solved by a process for the precipitation, crystallization, re-crystallization or re-slurry of intermediate of formula (II) as outlined in the annexed claims, whose definitions are integral part of the present description.

Particularly, the present invention provides a process for the preparation of Indomethacin of formula (I): 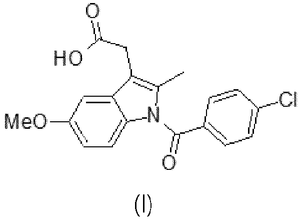 by means of precipitation, crystallization, re-crystallization or re-slurry of intermediate of formula (II): 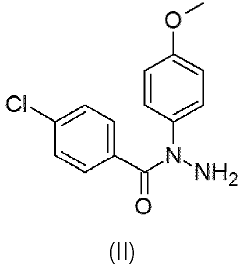 in a mixture comprising tetrahydrofuran and a C₁-C₄ linear or branched alkyl alcohol, followed by conversion of the obtained compound of formula (II) to give Indomethacin.

As a further aspect, the invention provides a process for the preparation of intermediate of formula (II) characterized by high purity as determined by HPLC.

As a further aspect, the present invention provides a process for the purging of process impurities of formula (III) and formula (IV) from intermediate of formula (II).

As another aspect, it is provided a process to prepare intermediate of formula (II) having impurity (III) content below 0.15% A/A% as determined by HPLC and having impurity (IV) content below 0.15% A/A% as determined by HPLC.

Further features and advantages of the processes according to the invention will result from the description hereafter reported of examples of realization of the invention, provided as an indication of the invention.

### Description of embodiments

The present invention is related to a process for the preparation of Indomethacin of formula (I): 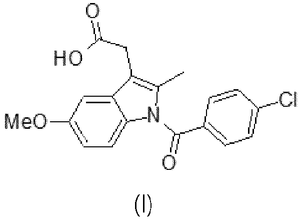 comprising the following steps:
i) precipitation, crystallization, re-crystallization or re-slurry of the compound 4-chloro-N-(4-methoxy phenyl)benzohydrazide of formula (II): 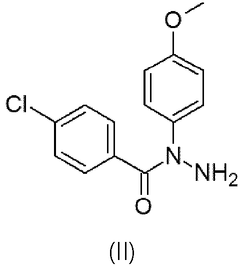 in a mixture comprising tetrahydrofuran and a C₁-C₄ linear or branched alkyl alcohol;
ii) conversion of compound of formula (II) obtained in step i)to give Indomethacin of formula (I):

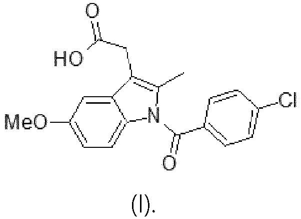

It was surprinsingly found, in fact, that by means of precipitation, crystallization, re-crystallization or re-slurry of intermediate of formula (II) in a mixture comprising tetrahydrofuran and a C₁-C₄ linear or branched alkyl alcohol, the quality of compound of formula (II) is significantly improved.

In particular, it was surprinsingly found that the content of impurity of formula (III) and the content of impurity of formula (IV) in compound of formula (II) is drastically lowered.

It follows that, by means of precipitation, crystallization, re-crystallization or re-slurry of intermediate of formula (II) in a mixture comprising tetrahydrofuran and a C₁-C₄ linear or branched alkyl alcohol, the quality of intermediate (II) and hence the quality of Indomethacin can be controlled.

By precipitation or crystallization it is intended the physical process of phase transformation of a dissolved substance into an insoluble solid, which remains suspended in the mixture. For promoting the precipitation or crystallization of a compound from a solution various methods are available, for example concentrating the solution until compound saturation is realized, cooling down said solution to lower compound solubility, adding a solvent in which the compound is poorly soluble or insoluble or seeding said solution with a small amount of the compound (seed). The solid obtained is then usually isolated by filtration or centrifugation.

Suspension means a solid material suspended in a solvent or solution, i.e. mixture of a solid with a solvent, which is liquid.

Solution means a liquid mixture in which the minor components (the solute) is dissolved and uniformly distributed within the major component (the solvent).

By re-crystallization it is intended that a solid compound is dissolved in a solvent and then precipitated for example by cooling the solution and/or adding another solvent. The solid obtained is then usually isolated by filtration or centrifugation.

By re-slurry it is intended that a solid compound is suspended in a solvent or in mixture of solvents. The solid obtained is then usually isolated by filtration or centrifugation.

According to a preferred embodiment of the process of the present invention, the term C₁-C₄ linear or branched alkyl alcohol, a component of the above mentioned mixture, means one alkyl alcohol selected in the group comprising: Methanol; Ethanol; 1-Propanol (i.e. Propanol); 2-Propanol (i.e. Isopropanol); 1-Butanol (i.e. Butanol); 2-Butanol (i.e. sec-Butanol); 2-methyl-1-Propanol (i.e. iso-Butanol); 2-methyl-2-Propanol (i.e. tert-Butanol).

According to a more preferred embodiment of the process of the present invention, the alkyl alcohol is isopropanol.

According to a preferred embodiment of the process of the present invention, the ratio between tetrahydrofuran and the C₁-C₄ linear or branched alkyl alcohol is comprised in the range from 4:1 to 1:4 v/v.

According to a more preferred embodiment of the process of the present invention, the ratio between tetrahydrofuran and the C₁-C₄ linear or branched alkyl alcohol is comprised in the range from 2:1 to 1:2 v/v.

According to a even more preferred embodiment of the process of the present invention, the ratio between tetrahydrofuran and the C₁-C₄ linear or branched alkyl alcohol is 1:1 v/v.

As intended herein, the expression v/v is the abbreviation of volume per volume, thus, for example, the proportion of two solvents within a mixture as measured by the volume occupied by each solvent. A mixture of tetrahydrofuran/isopropanol 2/1 v/v means, for example, 2 Liters of tetrahydrofuran per 1 Liter of isopropanol.

According to a preferred embodiment of the process of the present invention for preparing Indomethacin (I), in step i) the following steps are comprised:
a) charging 4-chloro-N-(4-methoxyphenyl)benzohydrazide of formula (II) and tetrahydrofuran;
b) heating the mixture of step a) until complete dissolution of the compound of formula (II);
c) cooling down the mixture of step b) to a temperature comprised in the range from 35°C to 60°C over a period comprised in the range from 30 minutes to 2 hours;
d) dosing the C₁-C₄ linear or branched alkyl alcohol into the mixture of step c) over a period comprised in the range from 30 minutes to 3 hours at a temperature comprised in the range from 35°C to 60°C;
e) stirring the mixture of step d) for a period comprised in the range from 30 minutes to 3 hours at a temperature comprised in the range from 35°C to 60°C;
f) cooling down the mixture of step e) to a temperature comprised in the range from -15°C to 30°C over a period comprised in the range from 30 minutes to 5 hours;
g) stirring the mixture of step f) at a temperature comprised in the range from -15°C to 30°C for a period comprised in the range from 30 minutes to 5 hours;
h) isolating the solid obtained in step g) by filtration or centrifugation to obtain 4-chloro-N-(4-methoxyphenyl)benzo hydrazide of formula (I).

According to a preferred embodiment of the process of the present invention, in step a) tetrahydrofuran is charged from 1 volume to 10 volumes with respect to 4-chloro-N-(4-methoxyphenyl)benzohydrazide of formula (II).

According to a more preferred embodiment of the process of the present invention, in step a) tetrahydrofuran is charged from 1 volume to 5 volumes with respect to 4-chloro-N-(4-methoxyphenyl)benzohydrazide of formula (II).

According to a even more preferred embodiment of the process of the present invention, in step a) tetrahydrofuran is charged from 1 volume to 2 volumes with respect to 4-chloro-N-(4-methoxyphenyl)benzo hydrazide of formula (II).

The term "volume" means volume of solvent per unit of product, thus, for example, 1 volume is 1 Liter per 1 Kilo, or 1 mL per 1 gram, or 1 microliter per 1 milligram. Thus, 10 volumes means for example 10 liters per 1 Kilogram of substance.

According to a preferred embodiment of the process of the present invention, in step c) the mixture is cooled down to a temperature comprised in the range from 49°C to 51°C over 1 hour.

According to a preferred embodiment of the process of the present invention, in step d) the alcohol is dosed from 1 volume to 10 volumes with respect to 4-chloro-N-(4-methoxyphenyl)benzohydrazide of formula (II).

According to a more preferred embodiment of the process of the present invention, in step d) the alcohol is dosed from 1 volume to 5 volumes with respect to 4-chloro-N-(4-methoxyphenyl)benzohydrazide of formula (II).

According to a even more preferred embodiment of the process of the present invention, in step d) the alcohol is dosed from 1 volume to 2 volumes with respect to 4-chloro-N-(4-methoxyphenyl)benzo hydrazide of formula (II).

According to a preferred embodiment of the process of the present invention, in step d) the the alcohol is dosed into the mixture over 1 hour at a temperature comprised in the range from 49°C to 51°C.

According to a preferred embodiment of the process of the present invention, in step e) the mixture is stirred for 1 hour at a temperature comprised in the range from 49°C to 51°C.

According to a preferred embodiment of the process of the present invention, in step f) the mixture is cooled down to a temperature comprised in the range from 4°C to 6°C over 2 hours.

According to a preferred embodiment of the process of the present invention, in step g) the mixture is stirred at a temperature comprised in the range from 4°C to 6°C for 1 hour.

According to a more preferred embodiment of the process of the present invention, in step c) the mixture is cooled down to a temperature comprised in the range from 49°C to 51°C over 1 hour, in step d) the the alcohol is dosed into the mixture over 1 hour at a temperature comprised in the range from 49°C to 51°C, in step e) the mixture is stirred for 1 hour at a temperature comprised in the range from 49°C to 51°C, in step f) the mixture is cooled down to a temperature comprised in the range from 4°C to 6°C over 2 hours and in step g) the mixture is stirred at a temperature comprised in the range from 4°C to 6°C for 1 hour.

According to a preferred embodiment of the process of the present invention for preparing Indomethacin (I), in step i) the following steps are comprised:
a1) charging 4-chloro-N-(4-methoxyphenyl)benzohydrazide of formula (I), tetrahydrofuran and a C₁-C₄ linear or branched alkyl alcohol;
b1) heating the mixture of step a1) to a temperature comprised in the range from 30°C to 70°C;
c1)stirring the mixture of step b1) at a temperature comprised in the range from 30°C to 70°C for a period comprised in the range from 1 hour to 5 hours;
d1) cooling down the mixture of step c1) to a temperature comprised in the range from 15°C to 35°C over a period comprised in the range from 10 minutes to 3 hours;
e1) stirring the mixture of step d1) at a temperature comprised in the range from 15°C to 35°C for a period comprised in the range from 10 minutes to 5 hours;
f1) isolating the solid obtained in step e1) by filtration or centrifugation to obtain 4-chloro-N-(4-methoxyphenyl)benzo hydrazide of formula (I).

According to a preferred embodiment of the process of the present invention, in step a1) tetrahydrofuran is charged from 1 volume to 10 volumes with respect to 4-chloro-N-(4-methoxyphenyl)benzohydrazide of formula (II) and the alcohol is charged from 1 volume to 10 volumes with respect to 4-chloro-N-(4-methoxyphenyl)benzohydrazide of formula (II).

According to a more preferred embodiment of the process of the present invention, in step a1) tetrahydrofuran is charged from 1 volume to 5 volumes with respect to 4-chloro-N-(4-methoxyphenyl)benzohydrazide of formula (II) and the alcohol is charged from 1 volume to 10 volumes with respect to 4-chloro-N-(4-methoxyphenyl)benzohydrazide of formula (II).

According to a even more preferred embodiment of the process of the present invention, in step a) tetrahydrofuran is charged from 1 volume to 2 volumes with respect to 4-chloro-N-(4-methoxyphenyl)benzo hydrazide of formula (II) and the alcohol is charged from 1 volume to 10 volumes with respect to 4-chloro-N-(4-methoxyphenyl)benzohydrazide of formula (II).

According to a preferred embodiment of the process of the present invention, in step b1) and step c1) the temperature is comprised in the range from 50°C to 55°C.

According to a preferred embodiment of the process of the present invention, in step d1) the mixture is cooled down to a temperature comprised in the range from 20°C to 25°C over 2 hours and in step e1) the mixture is stirred at a temperature comprised in the range from 20°C to 25°C for 1 hour.

According to a preferred embodiment of the process of the present invention, in step b1) and step c1) the temperature is comprised in the range from 50°C to 55°C, in step d1) the mixture is cooled down to a temperature comprised in the range from 20°C to 25°C over 2 hours and in step e1) the mixture is stirred at a temperature comprised in the range from 20°C to 25°C for 1 hour.

According to a preferred embodiment of the process of the present invention, step i) is repeated more than one time.

According to a preferred embodiment of the process of the present invention the compound 4-chloro-N-(4-methoxyphenyl)benzohydrazide of formula (II): 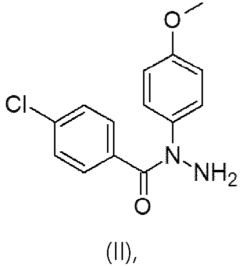 obtained in step i) has a content of impurity 4-chloro-N-(4-methoxyphenyl) benzamide of formula (III): 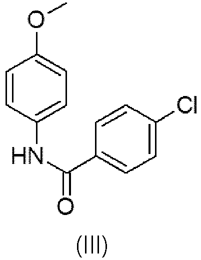 not higher than 0.15% A/A% as determined by HPLC, and a content of impurity 4-chloro-N'-(4-chlorobenzoyl)-N-(4-methoxyphenyl)benzo hydrazide of formula (IV): 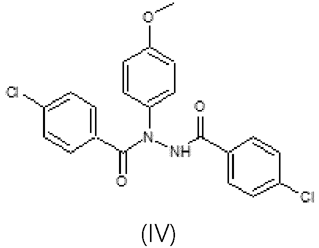 not higher than 0.15% A/A% as determined by HPLC.

As known by a person skilled in the art, in a HPLC chromatogram each peak represents one of the components found in the compound analyzed (sample). The area under each peak corresponds to the concentration of the corresponding component in the sample. By A/A% it is thus intended the concentration of each component as a percent of the total.

The present invention is also addressed to a process for the preparation of the compound 4-chloro-N-(4-methoxyphenyl) benzohydrazide of formula (II): 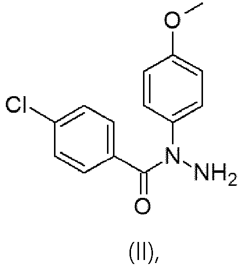 by precipitation, crystallization, re-crystallization or re-slurry of the compound 4-chloro-N-(4-methoxy phenyl)benzohydrazide of formula (II): 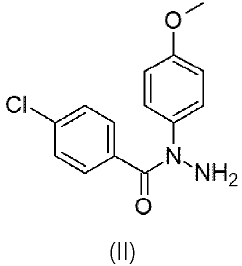 in a mixture comprising tetrahydrofuran and a C₁-C₄ linear or branched alkyl alcohol.

According to preferred embodiments of the present invention, the process for the preparation of compound of formula (II) is carried out applying one or more of the features described in the paragraphs above for step i).

Obviously and optionally, step i) of the process of the present invention can be re-applied on compound (II) to reach the desired purity of compound of formula (II) and/or Indomethacin of formula (I), specifically to reach the desired amount of impurity of formula (III) and impurity of formula (IV) in compound of formula (II) and/or Indomethacin of formula (I).

All the features and preferred embodiments of the process of the present invention given above can be combined in each possible combination to carry out the claimed process.

### Experimental Section

The starting material 4-chloro-N-(4-methoxyphenyl) benzohydrazide of formula (II) can be prepared according to well-known prior art methods, or for example, as described in J. Pharm. Biomed. Anal. 1998, 17, pages 409-413, or can be purchased on the market.

Room temperature (RT) means a temperature that is comprised in a range from 20 to 25°C, it is defined as comfortable temperature range indoors.

As intended herein, drying the wet material under *vacuum* means to heat the material to a given temperature for a given time while applying a reduced pressure. This operation is performed to remove residual solvents from the product.

### Example 1: Re-crystallization of compound of formula (II) in a mixture Tetrahydrofuran/Methanol 1:1 v/v.

In a round bottom flask equipped with a half-moon stirrer were charged: 20g of 4-chloro-N-(4-methoxyphenyl)benzohydrazide (compound of formula (II)), characterized by a content of impurity of formula (III) of 5.85% A/A% by HPLC and a content of impurity of formula (IV) of 11.36% A/A% by HPLC, 30 mL of methanol and 30 mL of tetrahydrofuran. The mixture was heated to T=50°C and kept stirring at this temperature for 1h. Thereafter, the mixture was cooled to RT over 2h and the resulting slurry was filtered. The wet solid was dried under *vacuum* at T=80°C for 12h. 11.2g of compound (II) were obtained, characterized by a content of impurity of formula (III) of 1.56% A/A% by HPLC and a content of impurity of formula (IV) of 2.55% A/A% by HPLC.

### Example 2: Re-crystallization of compound of formula (II) in a mixture Tetrahydrofuran/Ethanol 2:1 v/v.

In a round bottom flask equipped with a half-moon stirrer were charged: 20g of 4-chloro-N-(4-methoxyphenyl)benzohydrazide (compound of formula (II)), characterized by a content of impurity of formula (III) of 5.85% A/A% by HPLC and a content of impurity of formula (IV) of 11.36% A/A% by HPLC, 20 mL of ethanol and 40 mL of tetrahydrofuran. The mixture was heated to dissolution (T=55°C) and kept stirring at this temperature for 1h. Thereafter, the mixture was cooled to RT and stirred at this temperature for 1h. The slurry was then filtered, washing the cake with 10 mL of ethanol. The wet solid was dried under *vacuum* at T=55°C and 11.8g of compound (II) were obtained, characterized by a content of impurity of formula (III) of 0.38% A/A% by HPLC and a content of impurity of formula (IV) of 0.76% A/A% by HPLC.

### Example 3: Re-crystallization of compound of formula (II) in a mixture Tetrahydrofuran/Isopropanol 1:1 v/v.

In a round bottom flask equipped with a half-moon stirrer were charged: 100g of 4-chloro-N-(4-methoxyphenyl)benzohydrazide (compound of formula (II)), characterized by a content of impurity of formula (III) of 5.43% A/A% by HPLC and a content of impurity of formula (IV) of 11.39% A/A% by HPLC and 200 mL of tetrahydrofuran. The mixture was heated to dissolution (T=66°C) and kept stirring at this temperature for 20min. The mixture was cooled down to T=41°C over 1h. Thereafter, 100mL of isopropanol were dosed over 30min keeping the temperature within the range 41-41.5°C. The mixture was then heated to T=54-55°C and 100mL of isopropanol were added maintaining this temperature. After stirring at T=55°C for 1h, the mixture was cooled to T=21°C over 2h and then stirred at this temperature for 75min. The slurry was then filtered, washing the cake with 2x100 mL of isopropanol. The wet solid was dried under *vacuum* at T=80°C for 10h and 68.9g of compound (II) were obtained, characterized by a content of impurity of formula (III) of 0.59% A/A% by HPLC and a content of impurity of formula (IV) of 0.40% A/A% by HPLC.

### Example 4: Re-slurry of compound of formula (II) in a mixture Tetrahydrofuran/Isopropanol 1:1 v/v.

In a round bottom flask equipped with a half-moon stirrer were charged: 40g of 4-chloro-N-(4-methoxyphenyl)benzohydrazide (compound of formula (II)), characterized by a content of impurity of formula (III) of 5.43% A/A% by HPLC and a content of impurity of formula (IV) of 11.39% A/A% by HPLC and 80 mL of tetrahydrofuran. After 15min stirring at RT, 80 mL of isopropanol were added. The mixture was heated to T=51°C and the resulting suspension was kept stirring at this temperature for 1h. Thereafter, the mixture was cooled to RT over 2h and stirred at this temperature for 1h. The slurry was then filtered, washing the cake with 40 mL of isopropanol. The wet solid was dried under *vacuum* at T=80°C for 10h and 26.7g of compound (II) were obtained, characterized by a content of impurity of formula (III) of 0.62% A/A% by HPLC and a content of impurity of formula (IV) of 0.13% A/A% by HPLC.

### Example 5: Re-slurry of compound of formula (II) in a mixture Tetrahydrofuran/Isopropanol 1:1 v/v.

In a round bottom flask equipped with a half-moon stirrer were charged: 200g of 4-chloro-N-(4-methoxyphenyl)benzohydrazide (compound of formula (II)), characterized by a content of impurity of formula (III) of 1.48% A/A% by HPLC and a content of impurity of formula (IV) of 11.08% A/A% by HPLC and 400 mL of tetrahydrofuran. After 15min stirring at RT, 400 mL of isopropanol were added. The mixture was heated to T=53°C and the resulting suspension was kept stirring at this temperature for 1h. Thereafter, the mixture was cooled to T=22°C over 2h and stirred at this temperature for 65min. The slurry was then filtered, washing the cake with 2x200 mL of isopropanol. The wet solid was dried under *vacuum* at T=80°C for 9.5h and 147g of compound (II) were obtained, characterized by a content of impurity of formula (III) of 0.10% A/A% by HPLC and a content of impurity of formula (IV) of 0.12% A/A% by HPLC.

### Example 6: Re-crystallization of compound of formula (II) in a mixture Tetrahydrofuran/Isopropanol 2:3 v/v.

In a 400 mL reactor equipped with an anchor stirrer were charged: 30g of 4-chloro-N-(4-methoxyphenyl)benzohydrazide (compound of formula (II)), characterized by a content of impurity of formula (III) of 1.61% A/A% by HPLC and a content of impurity of formula (IV) of 1.13% A/A% by HPLC and 60 mL of tetrahydrofuran. The mixture was heated to T=70°C until complete dissolution of the solid, then the mixture was cooled to T=50°C over 1h and 90mL of isopropanol were dosed over 90min keeping this temperature. After stirring at T=50°C for 30min, the mixture was cooled to T=-10°C over 180min and then stirred at this temperature for 1h. The slurry was then filtered, and the wet solid was dried under *vacuum* at T=80°C for 12h. 27.1g of compound (II) were obtained, characterized by a content of impurity of formula (III) of 0.06% A/A% by HPLC and a content of impurity of formula (IV) of 0.10% A/A% by HPLC.

### Example 7: Re-crystallization of compound of formula (II) in a mixture Tetrahydrofuran/Isopropanol 2:3 v/v.

In a 400 mL reactor equipped with an anchor stirrer were charged: 30g of 4-chloro-N-(4-methoxyphenyl)benzohydrazide (compound of formula (II)), characterized by a content of impurity of formula (III) of 1.61% A/A% by HPLC and a content of impurity of formula (IV) of 1.13% A/A% by HPLC and 60 mL of tetrahydrofuran. The mixture was heated to T=70°C until complete dissolution of the solid, then the mixture was cooled to T=50°C over 1h and 90mL of isopropanol were dosed over 30min keeping this temperature. After stirring at T=50°C for 90min, the mixture was cooled to T=-10°C over 180min and then stirred at this temperature for 1h. The slurry was then filtered, and the wet solid was dried under *vacuum* at T=80°C for 12h. 28.8g of compound (II) were obtained, characterized by a content of impurity of formula (III) of 0.06% A/A% by HPLC and a content of impurity of formula (IV) of 0.15% A/A% by HPLC.

### Example 8: Re-crystallization of compound of formula (II) in a mixture Tetrahydrofuran/Isopropanol 2:1 v/v.

In a 400 mL reactor equipped with an anchor stirrer were charged: 30g of 4-chloro-N-(4-methoxyphenyl)benzohydrazide (compound of formula (II)), characterized by a content of impurity of formula (III) of 1.61% A/A% by HPLC and a content of impurity of formula (IV) of 1.13% A/A% by HPLC and 60 mL of tetrahydrofuran. The mixture was heated to T=70°C until complete dissolution of the solid, then the mixture was cooled to T=50°C over 1h and 30mL of isopropanol were dosed over 30min keeping this temperature. After stirring at T=50°C for 90min, the mixture was cooled to T=-10°C over 60min and then stirred at this temperature for 1h. The slurry was then filtered, and the wet solid was dried under *vacuum* at T=80°C for 12h. 26.5g of compound (II) were obtained, characterized by a content of impurity of formula (III) of 0.09% A/A% by HPLC and a content of impurity of formula (IV) of 0.12% A/A% by HPLC.

### Example 9: Re-crystallization of compound of formula (II) in a mixture Tetrahydrofuran/Isopropanol 2:3 v/v.

In a 400 mL reactor equipped with an anchor stirrer were charged: 30g of 4-chloro-N-(4-methoxyphenyl)benzohydrazide (compound of formula (II)), characterized by a content of impurity of formula (III) of 1.61% A/A% by HPLC and a content of impurity of formula (IV) of 1.13% A/A% by HPLC and 60 mL of tetrahydrofuran. The mixture was heated to T=70°C until complete dissolution of the solid, then the mixture was cooled to T=50°C over 1h and 90mL of isopropanol were dosed over 90min keeping this temperature. After stirring at T=50°C for 30min, the mixture was cooled to T=20°C over 60min and then stirred at this temperature for 1h. The slurry was then filtered, and the wet solid was dried under *vacuum* at T=80°C for 12h. 27.7g of compound (II) were obtained, characterized by a content of impurity of formula (III) of 0.08% A/A% by HPLC and a content of impurity of formula (IV) of 0.14% A/A% by HPLC.

### Example 10: Re-crystallization of compound of formula (II) in a mixture Tetrahydrofuran/Isopropanol 1:1 v/v.

In a 400 mL reactor equipped with an anchor stirrer were charged: 30g of 4-chloro-N-(4-methoxyphenyl)benzohydrazide (compound of formula (II)), characterized by a content of impurity of formula (III) of 1.61% A/A% by HPLC and a content of impurity of formula (IV) of 1.13% A/A% by HPLC and 60 mL of tetrahydrofuran. The mixture was heated to T=70°C until complete dissolution of the solid, then the mixture was cooled to T=50°C over 1h and 60mL of isopropanol were dosed over 60min keeping this temperature. After stirring at T=50°C for 60min, the mixture was cooled to T=5°C over 120min and then stirred at this temperature for 1h. The slurry was then filtered, and the wet solid was dried under *vacuum* at T=80°C for 12h. 25.6g of compound (II) were obtained, characterized by a content of impurity of formula (III) of 0.09% A/A% by HPLC and a content of impurity of formula (IV) of 0.12% A/A% by HPLC.

### Example 11: Comparative example (not of invention). Re-crystallization of compound of formula (II) in Isopropanol.

In a round bottom flask equipped with an half-moon stirrer were charged: 5g of 4-chloro-N-(4-methoxyphenyl)benzohydrazide (compound of formula (II)), characterized by a content of impurity of formula (III) of 5.85% A/A% by HPLC and a content of impurity of formula (IV) of 11.36% A/A% by HPLC and 20 mL of isopropanol. The mixture was heated to reflux until complete dissolution of the solid and the mixture was stirred at this temperature for 45min. After cooling down to RT over 120min, the mixture was stirred at this temperature for further 2h. The slurry was then filtered, and the wet solid was dried under *vacuum* at T=60°C for 11h. 4.5g of compound (II) were obtained, characterized by a content of impurity of formula (III) of 5.65% A/A% by HPLC and a content of impurity of formula (IV) of 8.19% A/A% by HPLC.

### Example 12: Comparative example (not of invention). Re-crystallization of compound of formula (II) in Tetrahydrofuran.

In a round bottom flask equipped with an half-moon stirrer were charged: 5g of 4-chloro-N-(4-methoxyphenyl)benzohydrazide (compound of formula (II)), characterized by a content of impurity of formula (III) of 5.85% A/A% by HPLC and a content of impurity of formula (IV) of 11.36% A/A% by HPLC and 20 mL of tetrahydrofuran. The mixture was heated to T=60°C until complete dissolution of the solid and the mixture was stirred at this temperature for 30min. After cooling down to RT over 120min, the mixture was stirred at this temperature for further 90min. The slurry was then filtered, and the wet solid was dried under *vacuum* at T=80°C for 8h. 2.7g of compound (II) were obtained, characterized by a content of impurity of formula (III) of 2.73% A/A% by HPLC and a content of impurity of formula (IV) of 5.16% A/A% by HPLC.

### Example 13: Table comparing the results of the Experiments reported above

| **Example n°** | **Starting material Compound (II)** | | **Type of experiment** | **Solvent** | **Product Compound (II)** | |
|---|---|---|---|---|---|---|
| | **Imp. (III) (HPLC A/A%)** | **Imp. (IV) (HPLC A/A%)** | | | **Imp. (III) (HPLC A/A%)** | **Imp. (IV) (HPLC A/A%)** |
| Example 1 | 5.85 | 11.36 | Recrystallization | THF/MeOH 1:1 | 1.56 | 2.55 |
| Example 2 | 5.85 | 11.36 | Recrystallization | THF/EtOH 2:1 | 0.38 | 0.76 |
| Example 3 | 5.43 | 11.39 | Recrystallization | THF/IPA 1:1 | 0.59 | 0.40 |
| Example 4 | 5.43 | 11.39 | Reslurry | THF/IPA 1:1 | 0.62 | 0.13 |
| Example 5 | 1.48 | 11.08 | Reslurry | THF/IPA 1:1 | 0.10 | 0.12 |
| Example 6 | 1.61 | 1.13 | Recrystallization | THF/IPA 2:3 | 0.06 | 0.10 |
| Example 7 | 1.61 | 1.13 | Recrystallization | THF/IPA 2:3 | 0.06 | 0.15 |
| Example 8 | 1.61 | 1.13 | Recrystallization | THF/IPA 2:1 | 0.09 | 0.12 |
| Example 9 | 1.61 | 1.13 | Recrystallization | THF/IPA 2:3 | 0.08 | 0.14 |
| Example 10 | 1.61 | 1.13 | Recrystallization | THF/IPA 1:1 | 0.09 | 0.12 |
| Example 11 (Comparative Ex) | 5.85 | 11.36 | Recrystallization | IPA | 5.65 | 8.19 |
| Example 12 (Comparative Ex) | 5.85 | 11.36 | Recrystallization | THF | 2.73 | 5.16 |

In the table above, tetrahydrofuran is reported as THF, methanol as MeOH, ethanol as EtOH and isopropanol as IPA.

The results of the Table above clearly show that using a single solvent, such as isopropanol or tetrahydrofuran (refer to Example 11 and Example 12) the rejection of impurity of formula (III) and impurity of formula (IV) is not very effective. On the contrary, when combining tetrahydrofuran and an alcohol as a solvent mixture, a very efficient impurities rejection is obtained. Such a Table clearly shows an unexpected synergic effect by combining said tetrahydrofuran and an alcohol.

### Example 14: Preparation of Indometacin (compound of formula (I)), starting from 4-chloro-N-(4-methoxyphenyl)benzohydrazide (compound of formula (II)) obtained from a re-crystallization or re-slurry process of compound of formula (II) in a mixture comprising tetrahydrofuran and a C₁-C₄ linear or branched alkyl alcohol.

In a 1000 mL reactor equipped with an anchor stirrer were charged: 50g of 4-chloro-N-(4-methoxyphenyl)benzohydrazide (compound of formula (II)) obtained following the procedure of Example 5) and 300 mL of toluene. The mixture was heated to T=75°C until complete dissolution of the solid, then the solvent was evaporated under *vacuum* until reaching 150 mL of residual volume. The residue was taken up with 150 mL of toluene and, keeping the temperature within T=65-68°C, 25g of levulinic acid were dosed over the mixture. After 2h stirring at T=65-68°C , 55.8 mL of phosphoric acid preheated to T=38-42°C were dosed over 30min while heating the mixture to T=100°C. After stirring at T=100°C for 60min, the mixture was cooled to T=75°C and 30 mL of water were added. After 30min at this temperature, the biphasic mixture was allowed to settle and the phases were separated. The aqueous layer was extracted at T=75°C with 100 mL of toluene and then again with 30 mL of toluene. The combined organic phases were washed at T=75°C with water (3x10 mL). Maintaining T=75°C, 1.9g of charcoal and 0.3g of dicalite were charged over the organic solution. After 15min stirring at this temperature, the mixture was filtered over a dicalite pad washing with 37 mL of toluene. The resulting solution was concentrated under *vacuum* to 120mL and then cooled down to T=20°C over 6h. The resulting slurry was filtered, washing the cake with toluene (20 mL). The wet solid was dried under *vacuum* at T=65°C for 14h. 49.6g of compound (I) were obtained, characterized by a content of impurity of formula (III) of 0.03% A/A% by HPLC and a content of impurity of formula (IV) of 0.11% A/A% by HPLC.

### Example 15: Analytical method for determining the chemical purity of 4-chloro-N-(4-methoxyphenyl)benzohydrazide (compound of formula (II)), via HPLC:

- Eclipse plus C8, 100 × 4.6 mm, 1.8 µm, or equivalent;
- Temp. Column: 55°C;
- Mobile Phase A: H₃PO₄ 0.1% / Acetonitrile 85/15 v/v;
- Mobile Phase B: H₃PO₄ 0.1% /Methanol/Acetonitrile 20/15/65 v/v;
- Gradient

| Time (min) | %A | %B |
|---|---|---|
| 0 | 100 | 0 |
| 4.0 | 65 | 35 |
| 6.5 | 50 | 50 |
| 10.5 | 0 | 100 |
| 11.0 | 0 | 100 |

- Flow: 2.0 mL/min;
- UV Detector: 235 nm;
- Injection Volume: 3 µL;
- Analysis Time: 11 min;
- Post-run Time: 2.5 min
- Diluent: Acetonitrile

### Example 16: Analytical method for determining the chemical purity of Crude Indomethacin (compound of formula (I)), via HPLC:

- Colum: EclipseXDB C18 , 50 × 4.6 mm, 1.8 µm, or equivalent;
- Temp. Column: 80°C;
- Mobile Phase A: H₃PO₄ 0.1% solution in water
- Mobile Phase B: H₃PO₄ 0.1% in Acetonitrile
- Gradient

| Time (min) | %A | %B |
|---|---|---|
| 0 | 70 | 30 |
| 2.5 | 20 | 80 |
| 5.0 | 20 | 80 |

- Analysis Time: 5 min;
- Post-run Time 2.5 min
- Flow: 1.0 mL/min;
- UV Detector: 235 nm;
- Injection Volume: 2 µL;
- Diluent: Acetonitrile.

## Claims

1. Process for the preparation of the Indomethacin compound of formula (I): 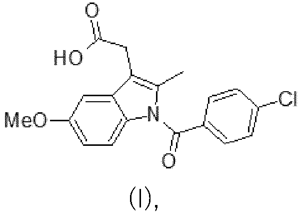 comprising the following steps:
i) precipitation, crystallization, re-crystallization or re-slurry of the compound 4-chloro-N-(4-methoxy phenyl)benzohydrazide of formula (II): 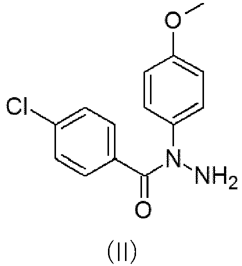 in a mixture comprising tetrahydrofuran and a C₁-C₄ linear or branched alkyl alcohol;
ii) conversion of compound of formula (II) obtained in step i) to give Indomethacin compound of formula (I):

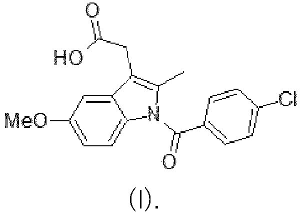

2. Process according to claim 1, wherein the C₁-C₄ alkyl alcohol is isopropanol.

3. Process according to any one of the claims from 1 to 2, wherein the ratio between tetrahydrofuran and the C₁-C₄ linear or branched alkyl alcohol is comprised in the range from 4:1 to 1:4 v/v.

4. Process according to claim 3, wherein the ratio between tetrahydrofuran and the C₁-C₄ linear or branched alkyl alcohol is comprised in the range from 2:1 to 1:2 v/v, or is 1:1 v/v.

5. Process according to any one of the claims from 1 to 4, wherein step i) comprises the following steps:
a) charging 4-chloro-N-(4-methoxyphenyl)benzohydrazide of formula (II) and tetrahydrofuran;
b) heating the mixture of step a) until complete dissolution of the compound of formula (II);
c) cooling down the mixture of step b) to a temperature comprised in the range from 35° C to 60° C over a period comprised in the range from 30 minutes to 2 hours;
d) dosing the C₁-C₄ linear or branched alkyl alcohol into the mixture of step c) over a period comprised in the range from 30 minutes to 3 hours at a temperature comprised in the range from 35° C to 60° C;
e) stirring the mixture of step d) for a period comprised in the range from 30 minutes to 3 hours at a temperature comprised in the range from 35° C to 60° C;
f) cooling down the mixture of step e) to a temperature comprised in the range from -15° C to 30° C over a period comprised in the range from 30 minutes to 5 hours;
g) stirring the mixture of step f) at a temperature comprised in the range from -15° C to 30° C for a period comprised in the range from 30 minutes to 5 hours;
h) isolating the solid obtained in step g) by filtration or centrifugation to provide 4-chloro-N-(4-methoxyphenyl)benzo hydrazide of formula (II).

6. Process according to claim 5, wherein in step c) the mixture is cooled down to a temperature comprised in the range from 49° C to 51° C over 1 hour.

7. Process according to any one of the claims from 5 to 6, wherein in step d) the alcohol is dosed into the mixture over 1 hour at a temperature comprised in the range from 49° C to 51° C.

8. Process according to any one of the claims from 5 to 7, wherein in step e) the mixture is stirred for 1 hour at a temperature comprised in the range from 49° C to 51° C, in step f) the mixture is cooled down to a temperature comprised in the range from 4° C to 6° C over 2 hours and in step g) the mixture is stirred at a temperature comprised in the range from 4° C to 6° C for 1 hour.

9. Process according to any one of the claims from 1 to 4, wherein step i) comprises the following steps:
a1) charging 4-chloro-N-(4-methoxyphenyl)benzohydrazide of formula (II), tetrahydrofuran and a C₁-C₄ linear or branched alkyl alcohol;
b1) heating the mixture of step a1) to a temperature comprised in the range from 30° C to 70° C;
c1) stirring the mixture of step b1) at a temperature comprised in the range from 30° C to 70° C for a period comprised in the range from 1 hour to 5 hours;
d1) cooling down the mixture of step c1) to a temperature comprised in the range from 15° C to 35° C over a period comprised in the range from 10 minutes to 3 hours;
e1) stirring the mixture of step d1) at a temperature comprised in the range from 15° C to 35° C for a period comprised in the range from 10 minutes to 5 hours;
f1) isolating the solid obtained in step e1) by filtration or centrifugation to provide 4-chloro-N-(4-methoxyphenyl)benzo hydrazide of formula (II).

10. Process according to claim 9, wherein in step b1) and step c1) the temperature is comprised in the range from 50° C to 55° C.

11. Process according to any one of the claims from 9 to 10 wherein in step d1) the mixture is cooled down to a temperature comprised in the range from 20° C to 25° C over 2 hours and in step e1) the mixture is stirred at a temperature comprised in the range from 20° C to 25° C for 1 hour.

12. Process according to any one of the claims from 1 to 11, wherein step i) is repeated more than one time.

13. Process according to any one of the claims from 1 to 12, wherein the compound 4-chloro-N-(4-methoxyphenyl)benzohydrazide of formula (II): 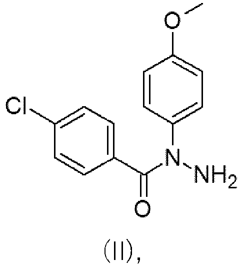 obtained in step i) has a content of impurity 4-chloro-N-(4-methoxyphenyl) benzamide of formula (III): 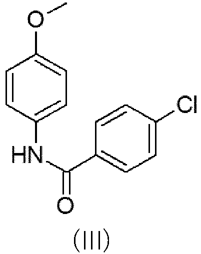 not higher than 0.15% A/A% as determined by HPLC, and a content of impurity 4-chloro-N'-(4-chlorobenzoyl)-N-(4-methoxyphenyl)benzo hydrazide of formula (IV): 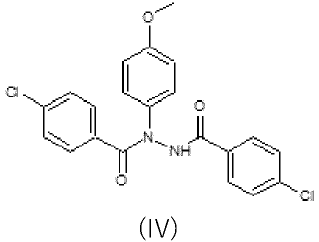 not higher than 0.15% A/A% as determined by HPLC.

14. Process for the preparation of the compound 4-chloro-N-(4-methoxyphenyl) benzohydrazide of formula (II): 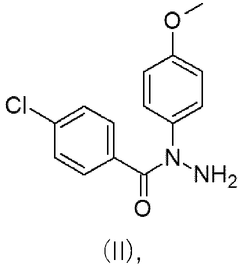 by precipitation, crystallization, recrystallization or re-slurry of the compound 4-chloro-N-(4-methoxy phenyl)benzohydrazide of formula (II): 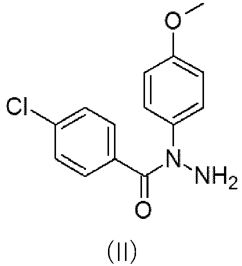 in a mixture comprising tetrahydrofuran and a C₁-C₄ linear or branched alkyl alcohol.

15. Process according to claim 14, comprising any one of the features claimed in any one of the claims from 2 to 13.

## Patentansprüche

1. Verfahren zur Herstellung der Verbindung Indometacin mit der Formel (I): 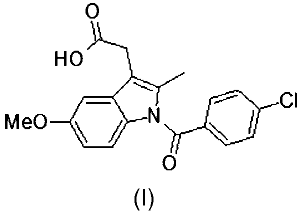 umfassend die folgenden Schritte:
i) Ausfällen, Kristallisieren, Umkristallisieren oder erneutes Aufschlämmen der Verbindung 4-Chlor-N-(4-methoxyphenyl)benzohydrazid mit der Formel (II): 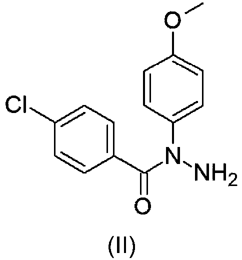 in einer Mischung, die Tetrahydrofuran und einen linearen oder verzweigten C₁-C₄-Alkylalkohol umfasst;
ii) Umwandeln der Verbindung mit der Formel (II), die in Schritt i) erhalten wurde, um die Verbindung Indometacin mit der Formel (I) zu ergeben:

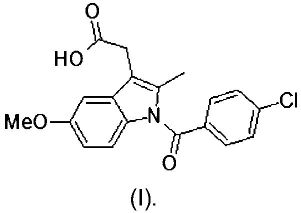

2. Verfahren nach Anspruch 1, wobei der C₁-C₄-Alkylalkohol Isopropanol ist.

3. Verfahren nach einem der Ansprüche 1 bis 2, wobei das Verhältnis zwischen Tetrahydrofuran und dem linearen oder verzweigten C₁-C₄-Alkylalkohol im Bereich von 4:1 bis 1:4 Vol./Vol. liegt.

4. Verfahren nach Anspruch 3, wobei das Verhältnis zwischen Tetrahydrofuran und dem linearen oder verzweigten C₁-C₄-Alkylalkohol im Bereich von 2:1 bis 1:2 Vol./Vol. liegt oder 1:1 Vol./Vol. ist.

5. Verfahren nach einem der Ansprüche 1 bis 4, wobei Schritt i) die folgenden Schritte umfasst:
a) Beschicken mit 4-Chlor-N-(4-methoxyphenyl)benzohydrazid mit der Formel (II) und Tetrahydrofuran;
b) Erwärmen der Mischung aus Schritt a), bis die Verbindung mit der Formel (II) vollständig gelöst ist;
c) Abkühlen der Mischung aus Schritt b) auf eine Temperatur, die im Bereich von 35 ° C bis 60 ° C liegt, über einen Zeitraum, der im Bereich von 30 Minuten bis 2 Stunden liegt;
d) Eindosieren des linearen oder verzweigten C₁-C₄-Alkylalkohols in die Mischung aus Schritt c) über einen Zeitraum, der im Bereich von 30 Minuten bis 3 Stunden liegt, bei einer Temperatur, die im Bereich von 35 ° C bis 60 ° C liegt;
e) Rühren der Mischung aus Schritt d) für einen Zeitraum, der im Bereich von 30 Minuten bis 3 Stunden liegt, bei einer Temperatur, die im Bereich von 35 ° C bis 60 ° C liegt;
f) Abkühlen der Mischung aus Schritt e) auf eine Temperatur, die im Bereich von -15 ° C bis 30 ° C liegt, über einen Zeitraum, der im Bereich von 30 Minuten bis 5 Stunden liegt;
g) Rühren der Mischung aus Schritt f) bei einer Temperatur, die im Bereich von -15 ° C bis 30 ° C liegt, für einen Zeitraum, der im Bereich von 30 Minuten bis 5 Stunden liegt;
h) Isolieren des Feststoffs, der in Schritt g) erhalten wurde, durch Filtration oder Zentrifugieren, um 4-Chlor-N-(4-methoxyphenyl)benzohydrazid mit der Formel (II) bereitzustellen.

6. Verfahren nach Anspruch 5, wobei in Schritt c) die Mischung über 1 Stunde auf eine Temperatur, die im Bereich von 49 ° C bis 51 ° C liegt, abgekühlt wird.

7. Verfahren nach einem der Ansprüche 5 bis 6, wobei in Schritt d) der Alkohol über 1 Stunde bei einer Temperatur, die im Bereich von 49 ° C bis 51 ° C liegt, in die Mischung eindosiert wird.

8. Verfahren nach einem der Ansprüche 5 bis 7, wobei in Schritt e) die Mischung 1 Stunde bei einer Temperatur, die im Bereich von 49 ° C bis 51 ° C liegt, gerührt wird, in Schritt f) die Mischung über 2 Stunden auf eine Temperatur abgekühlt wird, die im Bereich von 4 ° C bis 6 ° C liegt, und in Schritt g) die Mischung für 1 Stunde bei einer Temperatur gerührt wird, die im Bereich von 4 ° C bis 6 ° C liegt.

9. Verfahren nach einem der Ansprüche 1 bis 4, wobei Schritt i) die folgenden Schritte umfasst:
a1) Beschicken mit 4-Chlor-N-(4-methoxyphenyl)benzohydrazid mit der Formel (II), Tetrahydrofuran und einem linearen oder verzweigten C₁-C₄-Alkylalkohol;
b1) Erwärmen der Mischung aus Schritt a1) auf eine Temperatur, die im Bereich von 30 ° C bis 70 ° C liegt;
c1) Rühren der Mischung aus Schritt b1) bei einer Temperatur, die im Bereich von 30 ° C bis 70 ° C liegt, für einen Zeitraum, der im Bereich von 1 Stunde bis 5 Stunden liegt;
d1) Abkühlen der Mischung aus Schritt c1) auf eine Temperatur, die im Bereich von 15 ° C bis 35 ° C liegt, über einen Zeitraum, der im Bereich von 10 Minuten bis 3 Stunden liegt;
e1) Rühren der Mischung aus Schritt d1) bei einer Temperatur, die im Bereich von 15 ° C bis 35 ° C liegt, für einen Zeitraum, der im Bereich von 10 Minuten bis 5 Stunden liegt;
f1) Isolieren des Feststoffs, der in Schritt e1) erhalten wurde, durch Filtration oder Zentrifugieren, um 4-Chlor-N-(4-methoxyphenyl)benzohy-drazid mit der Formel (II) bereitzustellen.

10. Verfahren nach Anspruch 9, wobei in Schritt b1) und Schritt c1) die Temperatur im Bereich von 50 ° C bis 55 ° C liegt.

11. Verfahren nach einem der Ansprüche 9 bis 10, wobei in Schritt d1) die Mischung über 2 Stunden auf eine Temperatur abgekühlt wird, die im Bereich von 20 ° C bis 25 ° C liegt, und in Schritt e1) die Mischung für 1 Stunde bei einer Temperatur gerührt wird, die im Bereich von 20 ° C bis 25 ° C liegt.

12. Verfahren nach einem der Ansprüche 1 bis 11, wobei Schritt i) mehr als ein Mal wiederholt wird.

13. Verfahren nach einem der Ansprüche 1 bis 12, wobei die Verbindung 4-Chlor-N-(4-methoxyphenyl)benzohydrazid mit der Formel (II): 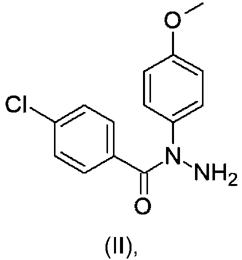 die in Schritt i) erhalten wurde, einen Gehalt an Verunreinigung 4-Chlor-N-(4-methoxyphenyl)benzamid mit der Formel (III): 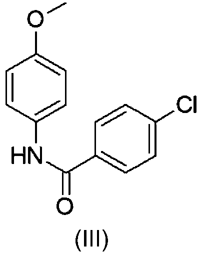 nicht höher als 0,15 % A/A %, bestimmt mittels HPLC, und einen Gehalt an Verunreinigung 4-Chlor-N'-(4-chlorbenzoyl)-N-(4-methoxyphenyl)benzohy-drazid mit der Formel (IV): 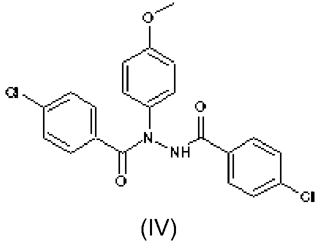 nicht höher als 0,15 % A/A %, bestimmt mittels HPLC, aufweist.

14. Verfahren zur Herstellung der Verbindung 4-Chlor-N-(4-methoxyphenyl)benzohydrazid mit der Formel (II): 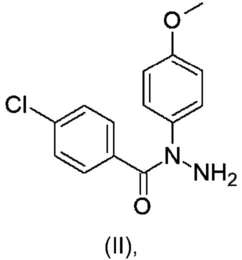 durch Ausfällen, Kristallisieren, Umkristallisieren oder erneutes Aufschlämmen der Verbindung 4-Chlor-N-(4-methoxyphenyl)benzohydrazid mit der Formel (II): 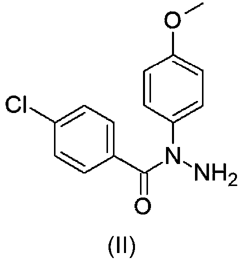 in einer Mischung, die Tetrahydrofuran und einen linearen oder verzweigten C₁-C₄-Alkylalkohol umfasst.

15. Verfahren nach Anspruch 14, umfassend jedwedes der Merkmale, die in einem der Ansprüche 2 bis 13 beansprucht wurden.

## Revendications

1. Procédé de préparation du composé indométhacine de formule (I) : 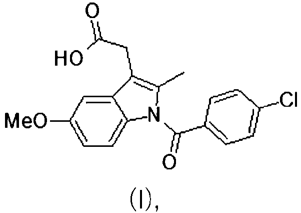 comprenant les étapes suivantes :
i) précipitation, cristallisation, recristallisation ou remise en suspension du composé 4-chloro-N-(4-méthoxyphényl)benzohydrazide de formule (II) : 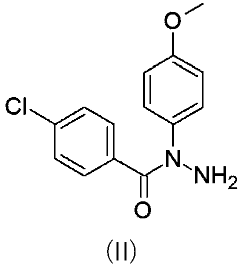 dans un mélange comprenant du tétrahydrofuranne et un alcool alkylique linéaire ou ramifié en C₁-C₄ ;
ii) conversion du composé de formule (II) obtenu à l'étape i) pour donner le composé indométhacine de formule (I) :

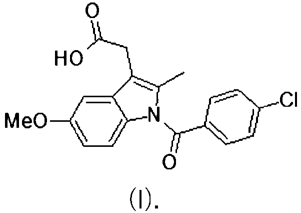

2. Procédé selon la revendication 1, dans lequel l'alcool alkylique en C₁-C₄ est l'isopropanol.

3. Procédé selon l'une quelconque des revendications 1 à 2, dans lequel le rapport entre le tétrahydrofuranne et l'alcool alkylique linéaire ou ramifié en C₁-C₄ est situé dans la plage de 4:1 à 1:4 V/V.

4. Procédé selon la revendication 3, dans lequel le rapport entre le tétrahydrofuranne et l'alcool alkylique linéaire ou ramifié en C₁-C₄ est situé dans la plage de 2:1 à 1:2 V/V ou est de 1:1 V/V.

5. Procédé selon l'une quelconque des revendications 1 à 4, dans lequel l'étape i) comprend les étapes suivantes :
a) chargement de 4-chloro-N-(4-méthoxyphényl)benzohydrazide de formule (II) et de tétrahydrofuranne ;
b) chauffage du mélange de l'étape a) jusqu'à dissolution complète du composé de formule (II) ;
c) refroidissement du mélange de l'étape b) à une température située dans la plage de 35 ° C à 60 ° C sur une période située dans la plage de 30 minutes à 2 heures ;
d) dosage de l'alcool alkylique linéaire ou ramifié en C₁-C₄ dans le mélange de l'étape c) sur une période située dans la plage de 30 minutes à 3 heures à une température située dans la plage de 35 ° C à 60 ° C ;
e) agitation du mélange de l'étape d) pendant une période située dans la plage de 30 minutes à 3 heures à une température située dans la plage de 35° C à 60 ° C ;
f) refroidissement du mélange de l'étape e) à une température située dans la plage de -15 ° C à 30 ° C sur une période située dans la plage de 30 minutes à 5 heures ;
g) agitation du mélange de l'étape f) à une température située dans la plage de -15 ° C à 30 ° C pendant une période située dans la plage de 30 minutes à 5 heures ;
h) isolement du solide obtenu à l'étape g) par filtration ou centrifugation afin d'obtenir le 4-chloro-N-(4-méthoxyphényl)benzohydrazide de formule (II).

6. Procédé selon la revendication 5, dans lequel, à l'étape c), le mélange est refroidi à une température située dans la plage de 49 ° C à 51 ° C en 1 heure.

7. Procédé selon l'une quelconque des revendications 5 à 6, dans lequel, à l'étape d), l'alcool est dosé dans le mélange en 1 heure à une température située dans la plage de 49 ° C à 51 ° C.

8. Procédé selon l'une quelconque des revendications 5 à 7, dans lequel, à l'étape e), le mélange est agité pendant 1 heure à une température située dans la plage de 49 ° C à 51 ° C, à l'étape f), le mélange est refroidi à une température située dans la plage de 4 ° C à 6 ° C en 2 heures et, à l'étape g), le mélange est agité à une température située dans la plage de 4 ° C à 6 ° C pendant 1 heure.

9. Procédé selon l'une quelconque des revendications 1 à 4, dans lequel l'étape i) comprend les étapes suivantes :
a1) chargement de 4-chloro-N-(4-méthoxyphényl)benzohydrazide de formule (II), de tétrahydrofuranne et d'un alcool alkylique linéaire ou ramifié en C₁-C₄ ;
b1) chauffage du mélange de l'étape a1) à une température située dans la plage de 30 ° C à 70 ° C ;
c1) agitation du mélange de l'étape b1) à une température située dans la plage de 30 ° C à 70 ° C pendant une période située dans la plage de 1 heure à 5 heures ;
d1) refroidissement du mélange de l'étape c1) à une température située dans la plage de 15 ° C à 35 ° C sur une période située dans la plage de 10 minutes à 3 heures ;
e1) agitation du mélange de l'étape d1) à une température située dans la plage de 15 ° C à 35 ° C pendant une période située dans la plage de 10 minutes à 5 heures ;
f1) isolement du solide obtenu à l'étape e1) par filtration ou centrifugation afin d'obtenir le 4-chloro-N-(4-méthoxyphényl)benzohydrazide de formule (II).

10. Procédé selon la revendication 9, dans lequel, à l'étape b1) et à l'étape c1), la température est située dans la plage de 50 ° C à 55 ° C.

11. Procédé selon l'une quelconque des revendications 9 à 10, dans lequel, à l'étape d1), le mélange est refroidi à une température située dans la plage de 20 ° C à 25 ° C en 2 heures et, à l'étape e1), le mélange est agité à une température située dans la plage de 20 ° C à 25 ° C pendant 1 heure.

12. Procédé selon l'une quelconque des revendications 1 à 11, dans lequel l'étape i) est répétée plus d'une fois.

13. Procédé selon l'une quelconque des revendications 1 à 12, dans lequel le composé 4-chloro-N-(4-méthoxyphényl)benzohydrazide de formule (II) : 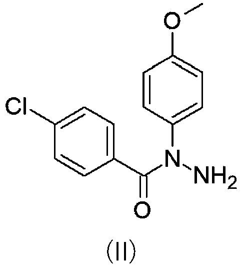 obtenu à l'étape i) présente une teneur en impureté 4-chloro-N-(4-méthoxyphényl)benzamide de formule (III) : 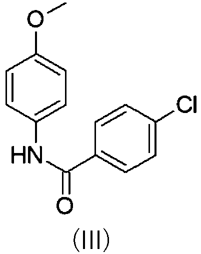 qui n'est pas supérieure à 0,15% A/A% telle que déterminée par HPLC et une teneur en impureté 4-chloro-N'-(4-chlorobenzoyl)-N-(4-méthoxyphényl)benzohydrazide de formule (IV) : 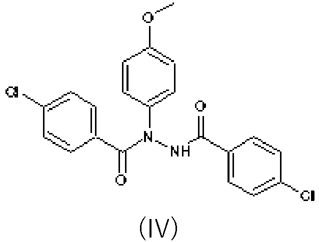 qui n'est pas supérieure à 0,15% A/A% telle que déterminée par HPLC.

14. Procédé de préparation du composé 4-chloro-N-(4-méthoxyphényl)benzohydrazide de formule (II) : 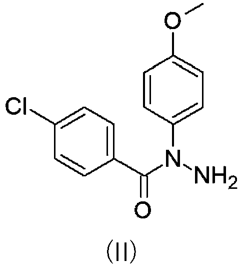 par précipitation, cristallisation, recristallisation ou remise en suspension du composé 4-chloro-N-(4-méthoxyphényl)benzohydrazide de formule (II) : 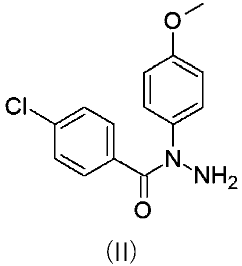 dans un mélange comprenant du tétrahydrofuranne et un alcool alkylique linéaire ou ramifié en C₁-C_{4.}

15. Procédé selon la revendication 14, comprenant l'une quelconque des caractéristiques revendiquées dans l'une quelconque des revendications 2 à 13.
